(19) Europäisches Patentamt
European Patent Office
Office européen des brevets

(11) Veröffentlichungsnummer : **0 418 990 B1**

(12) # EUROPÄISCHE PATENTSCHRIFT

(45) Veröffentlichungstag der Patentschrift : **10.11.93 Patentblatt 93/45**

(51) Int. Cl.$^5$ : **C07D 457/12**, A61K 31/435

(21) Anmeldenummer : **90250235.0**

(22) Anmeldetag : **13.09.90**

(54) **13-Brom und 13, 14-Dibrom-Ergoline, ihre Herstellung und Verwendung in Arzneimitteln.**

(30) Priorität : **20.09.89 DE 3931819**

(43) Veröffentlichungstag der Anmeldung : **27.03.91 Patentblatt 91/13**

(45) Bekanntmachung des Hinweises auf die Patenterteilung : **10.11.93 Patentblatt 93/45**

(84) Benannte Vertragsstaaten : **AT BE CH DE DK ES FR GB GR IT LI LU NL SE**

(56) Entgegenhaltungen :
**EP-A- 0 217 734**
**EP-A- 0 306 938**
**DE-A- 2 802 023**
**FR-A- 2 189 046**
**GB-A- 2 074 566**
**CHEMICAL ABSTRACTS, Band 112, 1990, Seite 764, Zusammenfassung Nr. 198878q, Columbus, Ohio, US; CS-A-262 283 (J. TAIMR et al.) 15-06-1989**

(73) Patentinhaber : **SCHERING AKTIENGESELLSCHAFT Berlin und Bergkamen**
**Müllerstrasse 170/178 Postfach 65 03 11**
**D-13303 Berlin (DE)**

(72) Erfinder : **Sauer, Gerhard, Dr.**
**Königsbacher Zeile 41A**
**D-1000 Berlin 28 (DE)**
Erfinder : **Brumby, Thomas, Dr.**
**Hauptstrasse 13**
**D-1000 Berlin 62 (DE)**
Erfinder : **Wachtel, Helmut, Dr.**
**Suarezstrasse 22**
**D-1000 Berlin 19 (DE)**
Erfinder : **Turner, Jonathan, Dr.**
**Ortwinstrasse 7**
**D-1000 Berlin 28 (DE)**
Erfinder : **Löschmann, Peter Andreas**
**Württembergallee 8**
**D-1000 Berlin 19 (DE)**

**EP 0 418 990 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft 13-Brom und 13,14-Dibrom Ergoline, ihre Herstellung und Verwendung in Arzneimitteln sowie Zwischenprodukte zur Herstellung derselben.

Aus DE-A-3824661.9 ist bekannt, daß längerkettige Kohlenwasserstoffreste in 6-Stellung die dopaminagonistische Aktivität von Ergolinen steigern. Diese Wirkungsweise wird überraschenderweise auch bei in Position 13 oder 13 und 14 bromierten Ergolinen beibehalten. Gleichzeitig wird die metabolische Stabilität der Verbindungen verbessert und damit eine erhöhte Bioverfügbarkeit erreicht.

Die Erfindung betrifft Verbindungen der Formel I

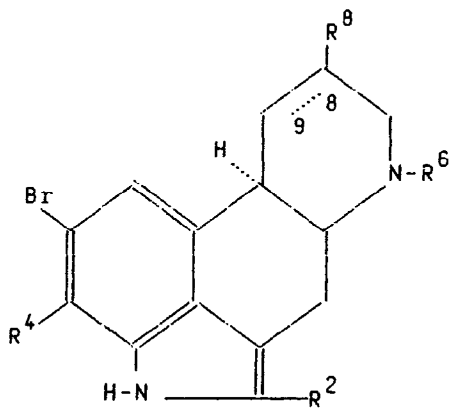

I

worin

| | |
|---|---|
| $R^2$ | $C_{1-6}$-Alkyl, $C_{2-6}$-Alkenyl oder $CH_2$-O-$C_{1-4}$-Alkyl, |
| $R^4$ | Wasserstoff oder Brom |
| $R^6$ | $C_{2-6}$-Alkyl, $C_{3-6}$-Alkenyl oder $C_{3-5}$-Cycloalkyl-$C_{1-2}$-Alkyl |
| $R^8$ | α-NH-CX-$R^3$, α-NH-$SO_2$-$NR^5R^7$, $CH_2$-Y, β-CO-NH-gg. substituiertes Phenyl, β-CO-$NR^9$-CO-$NHR^{10}$ |

worin

| | |
|---|---|
| X | Sauerstoff oder Schwefel, |
| $R^3$ | Wasserstoff, gegebenenfalls mit $C_{1-4}$-Alkoxy substituiertes $C_{1-6}$-Alkyl, -O-$(CH_2)_n$-$N(CH_3)_2$ oder -$N(C_{1-4}$-Alkyl$)_2$, |
| $R^5$ und $R^7$ | jeweils Wasserstoff oder $C_{1-4}$ -Alkyl, |
| Y | Wasserstoff, OH, O-$C_{1-6}$-Acyl, CN, $SCH_3$ oder $CONH_2$, der Substituent des Phenylrestes in o-, m- oder p-Stellung stehenden $C_{1-4}$-Alkyl, $C_{1-4}$-Alkoxy, F, Cl, Br oder J, |
| $R^9$ und $R^{10}$ | jeweils $C_{1-4}$-Alkyl oder -$(CH_2)_n$-$N(CH_3)_2$ bedeuten und n für 1, 2, 3 oder 4 steht und $C_8 \underline{...} C_9$ eine Einfach- oder Doppelbindung darstellt, wobei falls $R^8$ $CH_3$, $CH_2OH$ oder $CH_2$-O-$C_{1-6}$-Acyl bedeutet, $C_8 \underline{...} C_9$ eine Doppelbindung ist und falls $R^8$ $CH_2$-CN, $CH_2$-$SCH_3$ oder $CH_2$-$CONH_2$ bedeutet, $C_8 \underline{...} C_9$ eine Einfachbindung ist und $R^8$ β-ständig steht, sowie deren Säureadditionssalze. |

Unter Alkyl ist jeweils ein geradkettiger oder verzweigter Alkylrest zu verstehen wie beispielsweise Methyl, Ethyl, n-Propyl, Isopropyl, n-Butyl, Isobutyl, sek. Butyl, tert. Butyl, Pentyl, Hexyl, Heptyl, 2,2-Dimethylpropyl, 2-Methylbutyl, 3-Methylbutyl, 1-Ethylbutyl, Isopentyl, Isoheptyl, 1-Methyl-1-ethyl-propyl.

Bedeuten $R^2$ oder $R^6$ einen Alkenylrest, so enthält dieser bevorzugt nur eine Doppelbindung. wobei die Doppelbindung im Rest $R^6$ nicht benachbart vom Stickstoffatom stehen kann. Als Alkenylreste sind beispielsweise geeignet: Vinyl, 1-Propenyl, 2-Propenyl, 1-Methyl-2-propenyl, 1-Butenyl, Methallyl.

Als $C_{1-6}$-Acylgruppen sind aliphatische Carbonsäuren geeignet wie beispielsweise Ameisensaüre, Essigsäure, Propionsäure, Buttersäure, Capronsäure.

Der Substituent des Phenylrestes der in o-, m- oder p-Stellung stehen kann, ist $C_{1-4}$-Alkyl, $C_{1-4}$-Alkoxy oder Halogen wie Fluor, Chlor, Brom oder Jod.

Die Reste $R^9$ und $R^{10}$ stehen bevorzugt alternierend.

Als bevorzugte Ausführungsformen für $R^6$ und $R^2$ sind Kohlenwasserstoffe mit bis zu 4 C-Atomen anzusehen.

Die Verbindungen der Formel I können als E- oder Z-Isomere oder, falls ein chirales Zentrum im Rest $R^2$ vorhanden ist, als Diastereomere und als Gemische derselben auftreten. Die Isomeren und Isomerengemische sind von der vorliegenden Erfindung auch umfaßt. Die physiologisch verträglichen Säureadditionssalze leiten sich von den bekannten anorganischen und organischen Säuren ab wie zum Beispiel Salzsäure, Schwefelsäure, Bromwasserstoffsäure, Zitronensäure, Maleinsäure, Fumarsäure, Weinsäure u.a..

Die Verbindungen der Formel I sowie ihre Säureadditionssalze weisen insbesondere zentral dopaminerge Wirksamkeit auf und sind daher als Arzneimittel verwendbar.

Die dopaminagonistische Wirkung wurde mit Hilfe der von Horowski beschriebenen Methode der automatischen Registrierung von Stereotypien an Ratten bestimmt (Arzneim. Forsch. 12, 2281-2286, 1978): Unmittelbar nach intraperitonealer Prüfsubstanz- bzw. Vehikelverabreichung werden männliche Wistar-Ratten (90-120 g) einzeln in Zwangskäfige aus Acrylglas gesetzt. Über ein vor dem Kopf der Tiere angebrachtes elektrodynamisches Aufnahmesystem wird die Zahl der Kontakte an einem stählernen Becher mit einem zentralen Metallstab als Folge der stereotypen Kau-, Leck- und Nagebewegungen während 60 Minuten registriert. Die Mittelwerte ± S.E.M. der Anzahl der Kontakte während 60 Minuten für die verschiedenen Behandlungsgruppen, die mit jeweils 12 Tieren besetzt sind, werden berechnet und die Signifikanz der Unterschiede zwischen den Mittelwerten der verschiedenen Prüfsubstanzdosen im Vergleich zur Vehikel-behandelten Kontrollgruppe mit Hilfe der einfachen Varianzanalyse in Verbindung mit dem Dunnett-Test ermittelt.

Die Ergebnisse sind in der nachfolgenden Tabelle dargelegt.

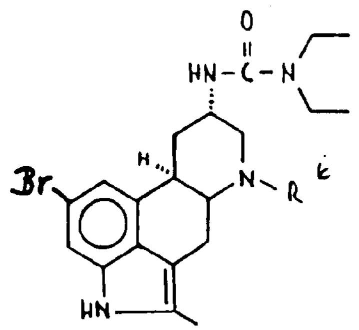

T A B E L L E

Auslösung von Stereotypien bei Ratten während 60 Minuten nach intraperitonealer Behandlung mit Vehikel bzw. verschiedenen Dosen von Ergolinharnstoff-Derivaten

(x: $p<0.05$, xx: $p<0.01$, Varianzanalyse/Dunnett-Test vs. Kontrolle; n: Anzahl der Tiere)

| | | Stereotypien (counts pro 60 Minuten) (Mittelwert ± S.E.M.) | | | | | | |
|---|---|---|---|---|---|---|---|---|
| | | | Prüfsubstanzdosis (mg/kg) | | | | | |
| $R^6$ | n | Kontrolle | 0.025 | 0.1 | 0.39 | 1.56 | 6.25 |
| | | | | | | | |
| $CH_2CH_3$ | 12 | 2137 ± 849 | 1943 ± 472 | 2470 ± 548 | 7499 ± 1342xx | 8066 ± 1248xx | 7381 ± 1336xx |

Da sich die erfindungsgemäßen Verbindungen insbesondere durch dopaminagonistische Wirkung auszeichnen, ohne daß starke $\alpha$-adrenerge Effekte auftreten, eignen sie sich insbesondere zur Behandlung des Morbus Parkinson.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel Wasser, Gelatine, Gummi arabicum, Milchzucker, Stärke, Magnesiumstearat, Talk, pflanzliche Ole, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Die erfindungsgemäßen Verbindungen werden in einer Dosis von 0,001 bis 10 mg aktiver Substanz in einen physiologisch verträglichen Träger eingebracht. Die Anwendung der erfindungsgemäßen Verbindungen erfolgt in einer Dosis von 0,00001 bis 0,1 mg/kg/Tag, vorzugsweise 0,001 bis 0,1 mg/kg/Tag analog dem bekannten Mittel Bromocryptin.

Die Herstellung der erfindungsgemäßen Verbindungen der Formel I kann nach an sich bekannten Methoden durchgeführt werden.

Beispielsweise gelangt man zu Verbindungen der Formel I gemäß Anspruch 1, indem man

a) eine Verbindung der Formel II

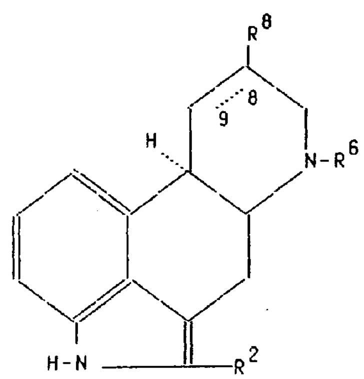

II

worin $R^6$, $R^8$ und $C_8\underline{...}C_9$ die obige Bedeutung haben und $R^2$ $C_{1-6}$-Alkyl ist, bromiert oder

b) eine Verbindung der Formel III oder deren Quartärsalz

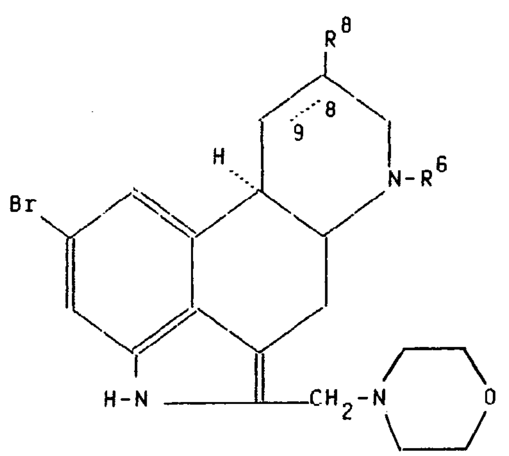

III

worin

$R^6$, $R^8$ und $C_8\underline{...}C_9$ die obige Bedeutung haben, in 2-Stellung substituiert oder

c) eine Verbindung der Formel IV

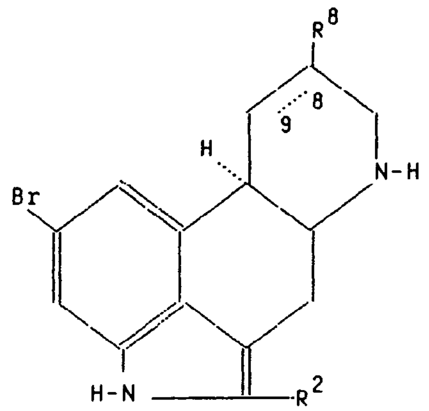

IV

worin $R^2$, $R^8$ und $C_8\underline{...}C_9$ die obige Bedeutung haben, alkyliert oder alkenyliert und gewünschtenfalls anschließend eine Carbonylgruppe thioliert und/oder die Säureadditionssalze bildet.

Die Verbindungen der Formel II werden nach Verfahren a) in stark saurer Lösung wie beispielsweise in Trifluoressigsäure oder Eisessig bromiert. Als Bromierungsmittel geeignet ist elementares Brom, Pyridin-hydrobromid-perbromid oder Pyrrolidon-hydro-perbromid, gegebenenfalls können chlorierte Kohlenwasserstoffe wie Chloroform, Methylenchlorid oder Ether wie Tetrahydrofuran, Dioxan und Isopropylether als Lösungsmittel zugesetzt werden. Die Bromierung wird bei Temperaturen von - 20 °C bis 80 °C, bevorzugt bei Raumtemperatur, durchgeführt und ist nach ca. 15 Minuten bis zu 1 Stunde beendet.

Werden molare Mengen Bromierungsreagenz zugesetzt, so erhält man hauptsächlich 13-Brom-Derivate; bei Einsatz von 2 Mol Bromierungsreagenz werden 13, 14-Di-brom-Derivate isoliert.

Die Einführung der Substituenten in 6-oder in 2-Stellung kann vor oder nach der Bromierung erfolgen. Nach dem Verfahren b) wird der Substituent $R^2$ nach dem in der Deutschen Patentanmeldung P 38 24 661.9 beschriebenen Verfahren eingeführt.

Hierbei wird die Mannichbase der Formel III nucleophil substituiert oder zum 2-Formyl-Derivat oxidiert, das anschließend in einer Wittig-Reaktion zur gewünschten Verbindung der Formel I umgesetzt wird.

Der nucleophile Austausch erfolgt gegebenenfalls nach Quartärnisierung der Aminomethylgruppe in einem inerten Lösungsmittel wie Alkoholen, polaren, aprotischen Lösungsmitteln, Ethern oder chlorierten Kohlenwasserstoffen bei Raumtemperatur oder erhöhter Temperatur, wobei als nucleophile Anionen Alkoholate eingesetzt werden können, die gewünschtenfalls anschließend in die $CH_2$-OH-Gruppe überführt werden können. Zur Herstellung des 2-Methyl-Derivates kann das Quartärsalz in polaren Lösungsmitteln wie Alkoholen mit Natriumborhydrid reduziert werden.

Die Oxidation zu einer 2-CHO-Verbindung kann analog dem in R.A. Jones et al. Synthetic Communications 16, 1799 (1986) beschriebenen Verfahren mit Braunstein oder tert. Butylhypochlorit in inerten Lösungsmitteln bei Raumtemperatur erfolgen. Die Umwandlung der 2-Formyl-Verbindungen zu Verbindungen der Formel I worin $R^2$ einen Alkenylrest bedeutet, kann in einer Wittigreaktion erfolgen, wie beispielsweise mit Alkyl-triphenylphosphonium-halogenid in polaren Lösungsmitteln wie cyclischen und acyclischen Ethern, chlorierten Kohlenwasserstoffen, Dimethylformamid oder Dimethylsulfoxid bei Temperaturen von -50 °C bis zur Siedetemperatur des Reaktionsgemisches, wobei zur Erzeugung des Ylens starke Basen wie Alkalialkoholate, Lithiumorganyl u.a. zugesetzt werden.

Enthält der Substituent $R^2$ eine Hydroxylgruppe, so kann diese beispielsweise durch Umsetzung mit Na $BH_4$ in Eisessig zum entsprechenden 2-Alkylderivat reduziert werden oder dehydratisiert werden unter Einführung einer Doppelbindung.

Die Darstellung von in 1-Stellung hydroxylierten Substituenten $R^2$ kann beispielsweise durch Grignardierung oder Lithium-alkylierung der 2-Aldehyde oder Ketone erfolgen. Die Grignardierung kann mit den üblichen Grignard-Reagenzien wie Alkylmagnesium-halogeniden in einem aprotischen Lösungsmittel wie cyclischen und acyclischen Ethern bei tiefen Temperaturen (-70 °C bis 0 °C) erfolgen. Die Umsetzung mit Alkyl-Lithium erfolgt unter analogen Bedingungen.

Die Substitution in 6-Stellung nach dem Verfahren c) kann beispielsweise nach A. Cerny et al. Coll. Czech. Chem. Comm. 49, 2828 (1984) oder nach dem in der EP-21206 beschriebenen Verfahren durchgeführt werden, indem man die 6H-Verbindung der Formel IV mit den entsprechenden $R^6$-Halogeniden (Bromiden, Chloriden, Iodiden) umsetzt. Zweckmäßigerweise erfolgt die Reaktion in einem inerten Lösungsmittel wie Dimethylsulfoxid, Dimethylformamid, Acetonitril oder Nitromethan in Gegenwart von Basen wie Alkalihydroxiden oder -carbonaten.

Die Uberführung der Amide und Harnstoffderivate in die Thioamide und Thioharnstoffderivate kann beispielsweise nach dem in der EP-A-217 730 beschriebenen Verfahren durch Umsetzung mit Phosphoroxychlorid und einem Thiolierungsmittel oder mit Lawesson-Reagenz nach Fieser and Fieser Reagents for Org. Synth. IX, 49 erfolgen. Die Verbindungen der Formel I werden entweder als freie Basen oder in Form ihrer physiologisch verträglichen Säureadditionssalze isoliert.

Zur Bildung von Salzen wird eine Verbindung der Formel I beispielsweise in wenig Methanol oder Methylenchlorid gelöst und mit einer konzentrierten Lösung der gewünschten Säure versetzt.

Die Isomerengemische können nach den üblichen Methoden wie beispielsweise Kristallisation, Chromatographie oder Salzbildung in die Diastereomeren bzw. E/Z-Isomeren aufgetrennt werden.

Die Erfindung umfaßt auch die Verbindungen der Formel III, die wertvolle Zwischenprodukte zur Herstellung pharmakologisch wirksamer Verbindungen darstellen. Die Umwandlung der Zwischenprodukte erfolgt nach den vorne beschriebenen Verfahren

Soweit die Herstellung der Ausgangsverbindungen nicht beschrieben wird, sind diese bekannt oder analog zu bekannten Verbindungen oder hier beschriebenen Verfahren herstellbar. Beispielsweise kann die Bromierung der Ausgangsverbindungen nach dem in der EP-A-217 734 beschriebenen Verfahren vorgenommen werden und die 2-Morpholinomethyl-gruppe nach der in DE-A-3824661.9 beschriebenen Methode eingeführt werden.

Die nachfolgenden Beispiele sollen das erfindungsgemäße Verfahren erläutern.

**Beispiel 1**

3-(13-Brom-2-methyl-6-n-propyl-8α-ergolinyl)-1,1-diethyl-harnstoff

Man löst 382 mg 1,1-Diethyl-3-(2-methyl-6-n-propyl-8α-ergolinyl)-harnstoff (1 mmol) in 20 ml Trifluoressigesäure und tropft bei Raumtemperatur 1 ml einer 1 molaren Lösung von Brom in Dichlormethan zu. Man rührt 30 Minuten, versetzt dann mit Eis, macht mit konz. Ammoniaklösung alkalisch und extrahiert mit Dichlormethan. Die organischen Phasen werden mit Natriumsulfat getrocknet und eingedampft. Der Rückstand wird an Kieselgel mit Dichlormethan/Methanol 95:5 chromatographiert. Ausbeute 259 mg (56 % der Theorie), nach Kristallisation aus Essigester 146 mg (31 % der Theorie), $[\alpha]_D$ = - 11° (0,5 % in Chloroform).

Auf analoge Weise werden aus den betreffenden Ausgangsverbindungen die folgenden 13-Brom-Derivate dargestellt:

3-(13-Brom-2-ethyl-6-n-propyl-8α-ergolinyl)-1,1-diethyl-harnstoff
Ausbeute 42 %
3-(13-Brom-6-ethyl-2-methyl-8α-ergolinyl)-1,1-diethyl-harnstoff
Ausbeute 26 %, $[\alpha]_D$ = - 17 ° (0,5 % in Chloroform)
N-(13-Brom-2-methyl-6-n-propyl-8α-ergolinyl)-formamid
Ausbeute 47 %
N-(13-Brom-2-ethyl-6-n-propyl-8α-ergolinyl)-formamid
Ausbeute 35 %
N-(13-Brom-2-methyl-6-n-propyl-8α-ergolinyl)-2-methyl-propionamid
Ausbeute 28 %, $[\alpha]_D$ = + 13° (0,5 % in Chloroform)
N-(13-Brom-2-methyl-6-n-propyl-8α-ergolinyl)-2-methyl-2-ethyl-butyrylamid
Ausbeute 47 %
N-(13-Brom-2-methyl-6-n-propyl-8α-ergolinyl)-methoxyacetamid
Ausbeute 32 %, $[\alpha]_D$ = + 7 ° (0,5 % in Chloroform)
N-(13-Brom-6-ethyl-2-methyl-8α-ergolinyl)-formamid
Ausbeute 48 %
3-(13-Brom-2-methyl-6-n-propyl-8α-ergolinyl)-1,1-diethyl-thioharnstoff
Ausbeute 14 %, $[\alpha]_D$ = + 20 ° (0,5 % in Chloroform)
(13-Brom-2-methyl-6-n-propyl-8α-ergolinylamino)-(dimethylamino)-sulfon
Ausbeute 41 %
13-Brom-2-methyl-8β-methylthiomethyl-6-n-propyl-ergolin
Ausbeute 16 %, $[\alpha]_D$ = - 46 ° (0,1 % in Chloroform)
13-Brom-6-cyclopropylmethyl-2-methyl-8β-methylthiomethyl-ergolin
Ausbeute 49 %, $[\alpha]_D$ = - 68 ° (0,1 % in Chloroform)
13-Brom-6-ethyl-2-methyl-8β-methylthiomethyl-ergolin
Ausbeute 29 %
13-Brom-6-cyclopropylmethyl-2-ethyl-8β-methylthiomethyl-ergolin

Ausbeute 31 %, $[\alpha]_D$ = - 60 ° (0,1 % in Chloroform)
13-Brom-2-ethyl-8β-methylthiomethyl-6-n-propyl-ergolin
Ausbeute 42 %, $[\alpha]_D$ = - 57 ° (0,1 % in Chloroform)
(13-Brom-2-methyl-6-n-propyl-8β-ergolinyl)-acetonitril
Ausbeute 65 %, $[\alpha]_D$ = - 42 ° (0,5 % in Chloroform)
(13-Brom-6-ethyl-2-methyl-8β-ergolinyl)-acetonitril
Ausbeute 47 %
(13-Brom-2-ethyl-6-n-propyl-8β-ergolinyl)-acetonitril
Ausbeute 40 %, $[\alpha]_D$ = - 40 ° (0,5 % in Chloroform)
(13-Brom-2-methyl-6-n-propyl-8β-ergolinyl)-acetamid
Ausbeute 36 %, $[\alpha]_D$ = - 47 ° (0,5 % in Pyridin)
(13-Brom-6-ethyl-2-methyl-8β-ergolinyll-acetamid
Ausbeute 52 %
(13-Brom-2-ethyl-6-n-propyl-8β-ergolinyl)-acetamid
Ausbeute 20 %, $[\alpha]_D$ = - 28 ° (0,1 % in Pyridin]
13-Brom-2-methyl-6-n-propyl-8β-ergolincarbonsäure-(4-fluoranilid)
Ausbeute 45 %, $[\alpha]_D$ = - 102 ° (0,1 % in Pyridin)
13-Brom-8,9-didehydro-2,8-dimethyl-6-n-propyl-ergolin
Ausbeute 38 %, $[\alpha]_D$ = - 51 ° (0,1 % in Chloroform)
13-Brom-8,9-didehydro-6-ethyl-2,8-dimethyl-ergolin
Ausbeute 11 %, $[\alpha]_D$ = - 36 ° (0,1 % in Chloroform)
13-Brom-8,9-didehydro-2,6-diethyl-8-methyl-ergolin
Ausbeute 19 %
13-Brom-8,9-didehydro-8-hydroxymethyl-2-methyl-6-n-propyl-ergolin
Ausbeute 43 %, $[\alpha]_D$ = - 51 ° (0,1 % in Pyridin]
13-Brom-8,9-didehydro-6-ethyl-8-hydroxymethyl-2-methyl-ergolin
Ausbeute 37 %

**Beispiel 2**

3-(13,14-Dibrom-2-methyl-6-n-propyl-8α-ergolinyl)-1,1-diethyl-harnstoff

Man löst 382 mg 1,1-Diethyl-3-(2-methyl-6-n-propyl-8α-ergolinyl)-harnstoff (1 mmol) in 20 ml Trifluoressigsäure und tropft bei Raumtemperatur 2 ml einer 1 molaren Lösung von Brom in Dichlormethan zu. Man rührt 30 Minuten, versetzt dann mit Eis, macht mit konz. Ammoniaklösung alkalisch und extrahiert mit Dichlormethan. Die organischen Phasen werden mit Natriumsulfat getrocknet und eingedampft. Der Rückstand wird an Kieselgel mit Dichlormethan/Methanol 95:5 chromatographiert, Ausbeute 56 %, $[\alpha]_D$ = - 14 ° (0,5 % in Chloroform)

Analog werden dargestellt:
3-(13,14-Dibrom-6-ethyl-2-methyl-8α-ergolinyl)-1,1-diethyl-harnstoff
Ausbeute 49 %, $[\alpha]_D$ = ± 0 ° (0,5 % in Chloroform)
N-(13,14-Dibrom-6-ethyl-2-methyl-8α-ergolinyl)-formamid
Ausbeute 63 %
N-(13,14-Dibrom-2-methyl-6-n-propyl-8α-ergolinyl)-methoxyacetamid
Ausbeute 67 %, $[\alpha]_D$ = + 2 ° (0,5 % in Chloroform)
13,14-Dibrom-6-ethyl-2-methyl-8β-methylthiomethyl-ergolin
Ausbeute 54 %
13,14-Dibrom-2-methyl-6-n-propyl-8β-methylthiomethyl-ergolin
Ausbeute 36 %
6-Cyclopropylmethyl-13,14-dibrom-2-ethyl-8β-methylthiomethyl-ergolin
Ausbeute 62 %
(13,14-Dibrom-6-ethyl-2-methyl-8β-ergolinyl)-acetamid
Ausbeute 43 %
13,14-Dibrom-8,9-didehydro-2,8-dimethyl-6-n-propyl-ergolin
Ausbeute 51 %

**Beispiel 3**

3-(13-Brom-6-n-propyl-2-vinyl-8α-ergolinyl)-1,1-diethyl-harnstoff

Man löst 4,47 g 3-(13-Brom-6-n-propyl-8α-ergolinyl)-1,1-diethyl-harnstoff (10 mmol), 8 g Morpholinhydrochlorid (63 mmol) und 1,5 g Paraformaldehyd (50 mmol) in 70 ml trockenem Dimethylformamid durch Erhitzen auf 100 °C. Nach 30 Minuten kühlt man ab, gießt die Mischung auf Eis, macht mit konz. Ammoniaklösung alkalisch und extrahiert mit Toluol. Die organischen Phasen werden mit Natriumsulfat getrocknet und eingedampft, der Rückstand in 30 ml Trifluoressigsäure gelöst und 30 Minuten auf 60 °C erwärmt. Diese Reaktionsmischung wird wieder auf Eis gegossen, man macht vorsichtig mit konz. Ammoniaklösung alkalisch und schüttelt mit Dichlormethan aus. Die organischen Phasen werden wie oben getrocknet und eingedampft, der Rückstand an Kieselgel mit Dichlormethan/Methanol chromatographiert. Man erhält 3,11 g öligen 3-(13-Brom-2-morpholino-methyl-6-n-propyl-8α-ergolinyl)-1,1-diethyl-harnstoff (57 % der Theorie). Dieses Rohprodukt wird in 150 ml Tetrahydrofuran gelöst, die Lösung auf - 40 °C abgekühlt, mit 3 ml Triethylamin versetzt und dann die Lösung von 1 ml tert.-Butylhypochlorit in 15 ml Tetrahydrofuran rasch zugetropft. Nach 30 Minuten Rühren gießt man die Mischung auf Eis, macht mit 25%igem Ammoniak alkalisch und schüttelt mit Essigester aus. Die organischen Phasen werden mit Natriumsulfat getrocknet und das Lösungsmittel abdestilliert, der Rückstand ist roher 3-(13-Brom-2-formyl-6-n-propyl-8α-ergolinyl)-1,1-diethyl-harnstoff, Ausbeute 2,0 g.
10 g Methyltriphenylphosphoniumbromid (28 mmol) suspendiert man in 100 ml wasserfreiem Tetrahydrofuran, kühlt auf - 70 °C ab und gibt 3,45 g Kalium-tert.-butylat zu. Nach 15 Minuten Rühren tropft man die Lösung des Rohproduktes in 50 ml wasserfreiem Tetrahydrofuran zu und läßt die Mischung in drei Stunden auf 0 °C erwärmen. Dann versetzt man mit gesättigter Kochsalzlösung und extrahiert mit Essigester. Die organischen Phasen werden getrocknet und eingedampft, der Rückstand an Kieselgel mit Dichlormethan/Methanol chromatographiert, Ausbeute 1,52 g. Durch Kristallisation aus Essigester/Diisopropylether werden 1,03 g des Endproduktes erhalten (21 % der Theorie).

Aus 3-(13-Brom-6-ethyl-8α-ergolinyl)-1,1-diethyl-harnstoff wird analog der 3-(13-Brom-6-ethyl-2-vinyl-8α-ergolinyl)-1,1-diethyl-harnstoff in 27 % Ausbeute dargestellt.

## Patentansprüche

1. Verbindungen der Formel I

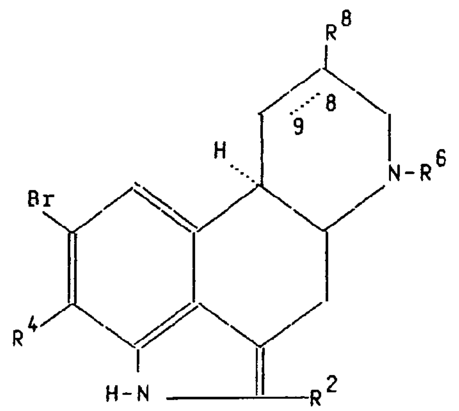

I

worin

$R^2$ $C_{1-6}$-Alkyl, $C_{2-6}$-Alkenyl oder $CH_2$-O-$C_{1-4}$-Alkyl

$R^4$ Wasserstoff oder Brom

$R^6$ $C_{2-6}$-Alkyl, $C_{3-6}$-Alkenyl oder $C_{3-5}$-Cycloalkyl-$C_{1-2}$-Alkyl

$R^8$ α-NH-CX-$R^3$, α-NH-$SO_2$-$NR^5R^7$, $CH_2$-Y, β-CO-NH-gg. substituiertes Phenyl, β-CO-$NR^9$-CO-$NHR^{10}$

worin

| | |
|---|---|
| X | Sauerstoff oder Schwefel, |
| $R^3$ | Wasserstoff, gegebenenfalls mit $C_{1-4}$-Alkoxy substituiertes $C_{1-6}$-Alkyl, -O-$(CH_2)_n$-$N(CH_3)_2$ oder -N($C_{1-4}$-Alkyl)$_2$, |
| $R^5$ und $R^7$ | jeweils Wasserstoff oder $C_{1-4}$-Alkyl, |
| Y | Wasserstoff, OH, O-$C_{1-6}$-Acyl, CN, $SCH_3$ oder $CONH_2$, der substituent des Phenylrestes ein in o-, m-, oder p-Stellung stehende $C_{1-4}$-Alkyl, $C_{1-4}$-Alkoxy, F, Cl, Br, oder J, |
| $R^9$ und $R^{10}$ | jeweils $C_{1-4}$-Alkyl oder -$(CH_2)_n$-$N(CH_3)_2$ bedeuten und n für 1, 2, 3 oder 4 steht und $C_8$...$C_9$ C eine Einfach- oder Doppelbindung darstellt, wobei falls $R^8$ $CH_3$, $CH_2OH$ oder $CH_2$-O-$C_{1-6}$-Acyl bedeutet, $C_8$...$C_9$ eine Doppelbindung ist und |

falls $R^8$ $CH_2$-CN, $CH_2$-$SCH_3$ oder $CH_2$-$CONH_2$ bedeutet, $C_8$...$C_9$ eine Einfachbindung ist und $R^8$ β-ständig steht, sowie deren Säureadditionssalze.

**2.** 3-(13-Brom-2-methyl-6-n-propyl-8α-ergolinyl)-1,1-diethyl-harnstoff
3-(13-Brom-6-ethyl-2-methyl-8α-ergolinyl)-1,1-diethyl-harnstoff
3-(13-Brom-2-methyl-6-n-propyl-8α-ergolinyl)-1,1-diethyl-thioharnstoff
3-(13,14-Dibrom-2-methyl-6-n-propyl-8α-ergolinyl)-1,1-diethyl-harnstoff
3-(13-Brom-6-n-propyl-2-vinyl-8α-ergolinyl)-1,1-diethyl-harnstoff

**3.** Arzneimittel auf Basis der Verbindungen nach Anspruch 1 und 2.

**4.** Verfahren zur Herstellung der Verbindungen nach Anspruch 1 dadurch, daß man
a) eine Verbindung der Formel II

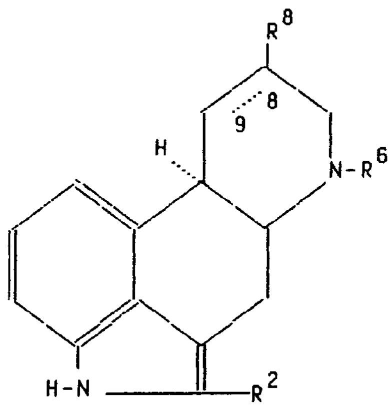

II

worin $R^6$, $R^8$ und $C_8$...$C_9$ die obige Bedeutung haben und $R^2$ $C_{1-6}$-Alkyl ist, bromiert oder
b) eine Verbindung der Formel III oder deren Quartärsalze

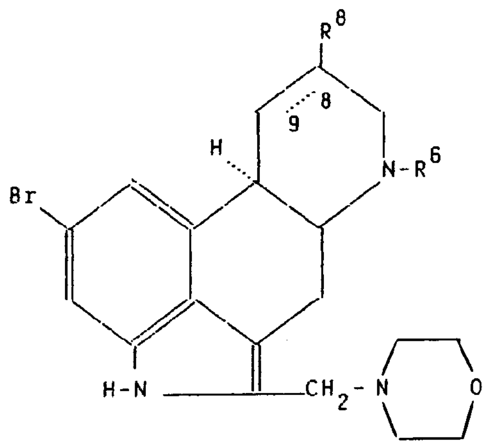

III

worin
$R^6$, $R^8$ und $C_8...C_9$ die obige Bedeutung haben, in 2-Stellung substituiert oder
c) eine Verbindung der Formel IV

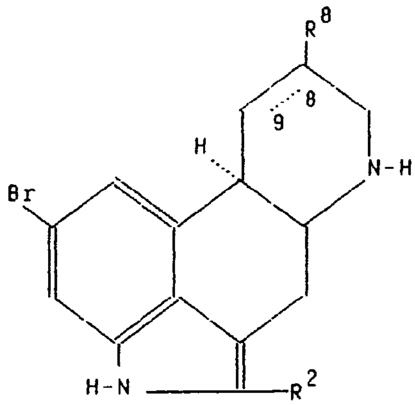

IV

worin $R^2$, $R^8$ und $C_8...C_9$ die obige Bedeutung haben, alkyliert oder alkenyliert und gewünschtenfalls anschließend eine Carbonylgruppe thioliert und/oder die Säureadditionssalze bildet.

**5.** Verbindungen der Formel III

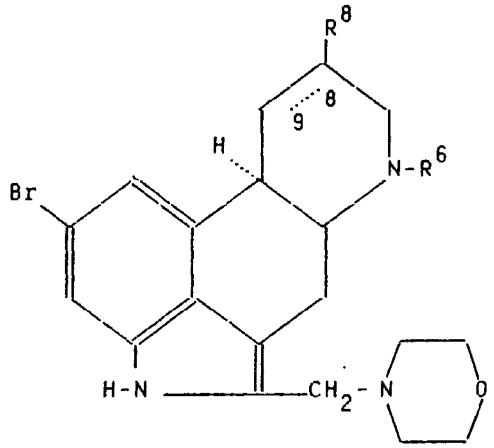

III

worin
$R^6$, $R^8$ und $C_8...C_9$ die obige Bedeutung haben.

## Claims

1. Compounds of formula I

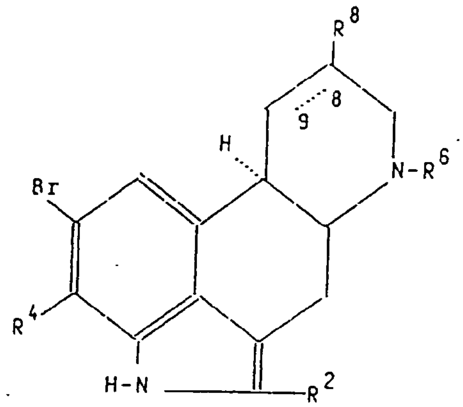

I

wherein

| | |
|---|---|
| $R^2$ | is $C_{1-6}$-alkyl, $C_{2-6}$-alkenyl or $CH_2$-O-$C_{1-4}$-alkyl, |
| $R^4$ | is hydrogen or bromine, |
| $R^6$ | is $C_{2-6}$-alkyl, $C_{3-6}$alkenyl or $C_{3-5}$-cycloalkyl-$C_{1-2}$-alkyl, |
| $R^8$ | is (α-NH-CX-$R^3$, α-NH-$SO_2$-$NR^5R^7$, $CH_2$-Y, β-CO-NH-, optionally substituted phenyl, β-CO-$NR^9$-CO-$NHR^{10}$, |

wherein

| | |
|---|---|
| X | is oxygen or sulphur, |
| $R^3$ | is hydrogen, optionally $C_{1-4}$-alkoxy-substituted $C_{1-6}$-alkyl, -O-$(CH_2)_n$-$N(CH_3)_2$ or -N($C_{1-4}$-alkyl)$_2$, each of $R^5$ and $R^7$ is hydrogen or $C_{1-4}$-alkyl, |
| Y | is hydrogen, OH, O-$C_{1-6}$-acyl, CN, $SCH_3$ or $CONH_2$, the substituent of the phenyl radical is $C_{1-4}$-alkyl, $C_{1-4}$-alkoxy, fluorine, chlorine, bromine or iodine, each of which is in the o-, m- or p-position, each of $R^9$ and $R^{10}$ is $C_{1-4}$-alkyl or -$(CH_2)_n$-$N(CH_3)_2$, and |
| n | stands for 1, 2, 3 or 4, and |
| $C_8$---$C_9$ | is a single or double bond, wherein, if $R^8$ is $CH_3$, $CH_2OH$ or $CH_2$-O-$C_{1-6}$-acyl, $C_8$---$C_9$ is a double bond, and, if $R^8$ is $CH_2$-CN, $CH_2$-$SCH_3$ or $CH_2$-$CONH_2$, $C_8$---$C_9$ is a single bond, and $R^8$ is in the β-configuration, |

as well as their acid addition salts.

2. 3-(13-bromo-2-methyl-6-n-propyl-8α-ergolinyl)-1,1-diethylurea,
3-(13-bromo-6-ethyl-2-methyl-8α-ergolinyl)-1,1-diethyl-urea,
3-(13-bromo-2-methyl-6-n-propyl-8α-ergolinyl)-1,1-diethylthiourea,
3-(13,14-dibromo-2-methyl-6-n-propyl-8α-ergolinyl)-1,1-diethylurea,
3-(13-bromo-6-n-propyl-2-vinyl-8α-ergolinyl)-1,1-diethyl-urea.

3. Medicaments based on the compounds according to claims 1 and 2.

4. Process for the preparation of the compounds according to claim 1 by
(a) brominating a compound of formula II

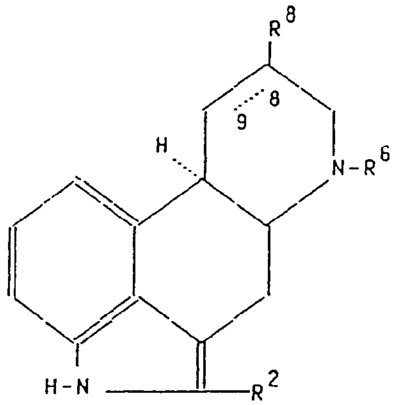

II

wherein
$R^6$, $R^8$ and $C_8$---$C_9$ have the above meanings and $R^2$ is $C_{1-6}$-alkyl, or
(b) substituting, in the 2-position, a compound of formula III or a quaternary salt thereof

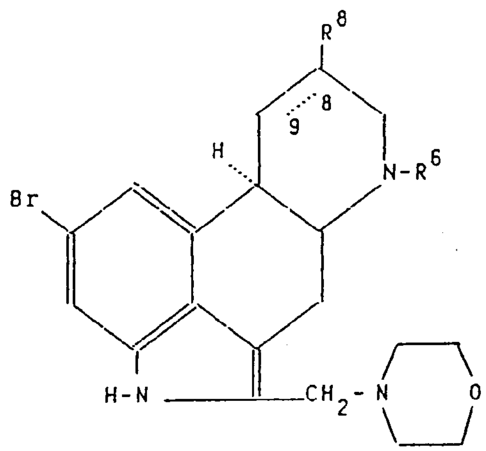

III

wherein $R^6$, $R^8$ and $C_8$---$C_9$ have the above meanings, or
(c) alkylating or alkenylating a compound of formula IV

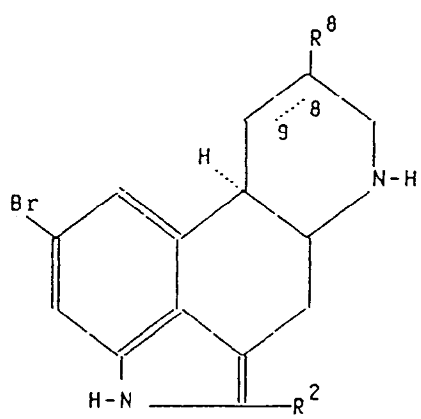

IV

wherein $R^2$, $R^8$ and $C_8$---$C_9$ have the above meanings, and, if desired, subsequently thiolating a carbonyl group and/or forming the acid addition salts.

**5.** Compounds of formula III

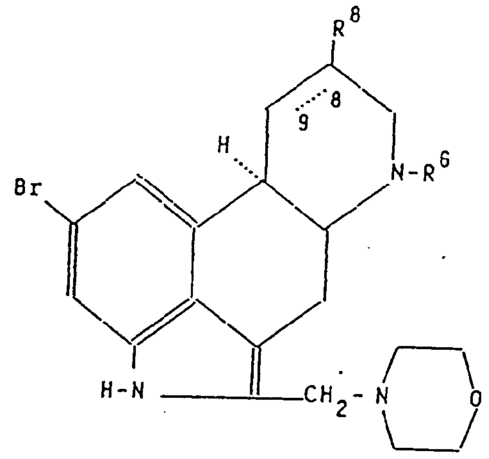

III

wherein $R^6$, $R^8$ and $C_8$---$C_9$ have the above meanings.

6. Process for the preparation of a medicament by mixing a compound of formula I prepared in accordance with claim 4 with a pharmaceutical carrier.

## Revendications

1. Composés de formule I

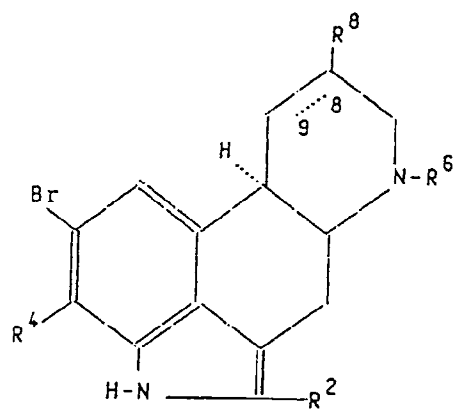

I

dans laquelle
$R^2$ est alkyle en $C_1$-$C_6$, alcényle en $C_2$-$C_6$, ou $CH_2$-O-(alkyle en $C_1$-$C_4$),
$R^4$ est l'hydrogène ou le brome,
$R^6$ est alkyle en $C_2$-$C_6$, alcényle en $C_3$-$C_6$ ou (cycloalkyl en $C_3$-$C_5$)-alkyle en $C_1$-$C_2$,
$R^8$ est α-NH-CX-$R^3$, α-NH-$SO_2$-$NR^5R^7$, $CH_2$-Y, β-CO-NH-phényle éventuellement substitué, β-CO-$NR^9$-CO-$NHR^{10}$,
où
X est l'oxygène ou le soufre,
$R^3$ est hydrogène, alkyle en $C_1$-$C_6$ éventuellement substitué par alcoxy en $C_1$-$C_4$, -O-$(CH_2)_n$-$N(CH_3)_2$ ou N(alkyle en $C_1$-$C_4$)$_2$,
$R^5$ et $R^7$ sont chacun hydrogène ou alkyle en $C_1$-$C_4$,
Y est hydrogène, OH, O-acyle en $C_1$-$C_6$, CN, $SCH_3$ ou $CONH_2$,
le substituant du reste phényle est alkyle en $C_1$-$C_4$, alcoxy en $C_1$-$C_4$, F, Cl, Br ou I, en position o, m ou p,
$R^9$ et $R^{10}$ sont chacun alkyle en $C_1$-$C_4$ ou -$(CH_2)_n$-$N(CH_3)_2$ et n est 1, 2, 3 ou 4, et $C_8$...$C_9$ représente une liaison simple ou double,
$C_8$...$C_9$ étant une liaison double au cas où $R^8$ est $CH_3$, $CH_2OH$ ou $CH_2$-O-(acyle en $C_1$-$C_6$) et
$C_8$...$C_9$ étant une liaison simple et $R^8$ étant en position β au cas où $R^8$ est $CH_2$-CN, $CH_2$-$SCH_3$ ou $CH_2$-$CONH_2$, et leurs sels d'addition d'acides.

**2.** 3-(13-bromo-2-méthyl-6-n-propyl-8α-ergolinyl)-1,1-diéthylurée
3-(13-bromo-6-éthyl-2-méthyl-8α-ergolinyl)-1,1-diéthylurée
3-(13-bromo-2-méthyl-6-n-propyl-8α-ergolinyl)-1,1-diéthyl-thiourée
3-(13,14-dibromo-2-méthyl-6-n-propyl-8α-ergolinyl)-1,1-diéthylurée
3-(13-bromo-6-n-propyl-2-vinyl-8α-ergolinyl)-1,1-diéthyl-urée

**3.** Médicaments à base des composés selon la revendication 1 et 2.

**4.** Procédé de préparation des composés selon la revendication 1, selon lequel
a) on brome un composé de formule II

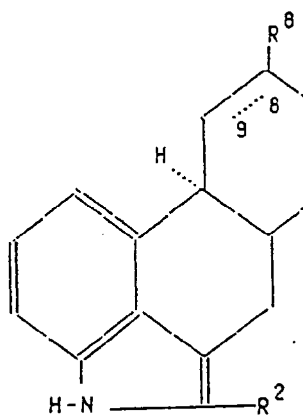

II

dans laquelle $R^6$, $R^8$ et $C_8\underline{...}C_9$ ont la signification donnée ci-dessus et $R^2$ est alkyle en $C_1$-$C_6$, ou
b) on substitue en position 2 un composé de formule III ou un de ses sels quaternaires

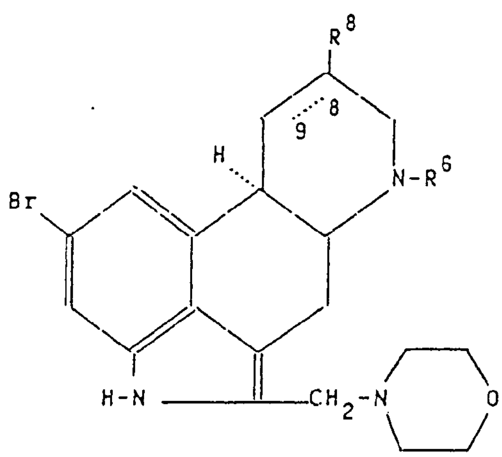

III

dans laquelle
$R^6$, $R^8$ et $C_8\underline{...}C_9$ ont la signification donnée ci-dessus, ou
c) on alkyle ou on alcényle un composé de formule IV

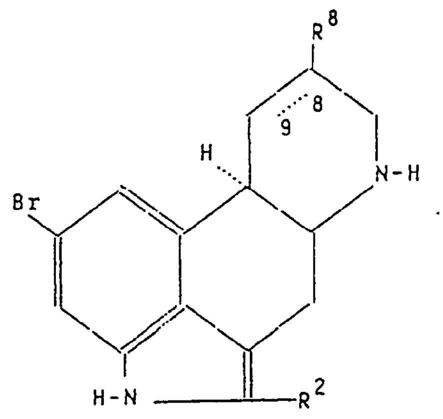

IV

dans laquelle $R^2$, $R^8$ et $C_8$...$C_9$ ont la signification donnée ci-dessus puis,si désiré, on thiole un groupe carboxy et/ou on forme les sels d'addition d'acides.

5. Composés de formule III

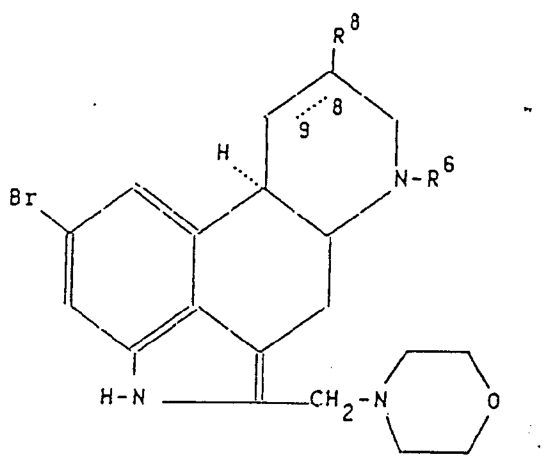

III

dans laquelle
$R^6$, $R^8$ et $C_8$...$C_9$ ont la signification donnée ci-dessus.

6. Procédé de préparation d'un médicament selon lequel on mélange un composé de formule I préparé selon la revendication 4 avec un support pharmaceutique.